# EUROPEAN PATENT APPLICATION

(11) **EP 1 949 890 A2**
(43) Date of publication of application: **30.07.2008**
(21) Application number: 08100711.4
(22) Date of filing: 31.05.2000
(51) Int. Cl.: A61K 9/06, A61K 9/22, A61K 47/34

(54) **Implantable gel compositions and method of manufacture**

(30) Priority: 04.06.1999 US 137815 P
(62) Divisional of application: 00939558.3
(71) Applicant: Alza Corporation, Mountain View, CA 94039-7210 (US)
(72) Inventor: Brodbeck, Kevin, J, California, CA 94043 (US); Prestrelski, Steven, J, California, CA 94043 (US); Pushpala, Shamim, J, California, CA 94087 (US)
(74) Representative: Williams, Paul Edwin

(57) **Abstract**

Methods and compositions for reducing the burst of beneficial agent from implantable systems is described. Such systems utilize compressed particulates of a beneficial agent, optionally mixed with a dissolution rate modulator or an agent exhibiting a characteristic of low solubility in water, such as a mixture of stearic acid and palmitic acid, dispersed throughout a bioerodible and biocompatible carrier.

## Description

### BACKGROUND OF THE INVENTION

### Field of the Invention

The present invention relates generally to implantable compositions that provide controlled release of a beneficial agent. In particular, the present invention relates to compositions of a carrier, such as a gel, and a beneficial agent in which the interaction or solubility of the beneficial agent with the gel components or an aqueous environment of use may be modulated by the bulk characteristics of the gel and the microenvironment associated with the beneficial agent. The invention also relates to methods of manufacturing compositions of the invention.

### Description of Related Art

Numerous systems have been described for the delivery of drugs and other beneficial agents from implantable polymeric matrices. Representative patents relating to such systems include, for example, U.S. Patent No. 5,085,866 describing a system for intraoral implantation. U.S. Patent No. 5,019,400 describes the preparation of controlled release microspheres; U.S. Patent No. 4,938,763 and its divisional U.S. Patent No. 5,278,201 describe, in-situ-forming, solid biodegradable implants; U.S. Patent 5,599,552 describes thermoplastic and thermoset polymer compositions that utilize solvents which are miscible to dispersible in water, such as N-methyl-2-pyrrolidone, resulting in polymer solutions capable of quickly absorbing water from surrounding tissue; U.S. Patent No. 5,242,910 describes a sustained release composition containing drugs for treating periodontal disease; U.S. Patent No. 5,620,700 describes a polymer-drug matrix, optionally including plasticizers in an amount up to about 30 wt %, for local application of drug in the peridontal cavity; and U.S. Patent No. 5,556,905 describes degradable thermoplastic compositions which are modified by plasticizers consisting of various partial esters of citric acid.

It has been well recognized that implantable systems often have difficulty delivering active agent, particularly active agent that is highly water soluble, in a controlled fashion during the time period immediately following implantation, often resulting in an undesirable "burst" effect that releases too much active agent immediately after implantation. Various compositions and methods have been described in the art to address the problem.

U.S. Patent No. 5,759,563 describes liquid delivery systems that may be used to form solid structures in which an active agent is incorporated into a controlled release component which is then dissolved, dispersed or entrained in the liquid component. As described, the controlled release component may include microstructures, macrostructures, conjugates, complexes or low water-solubility salts. The controlled release component is said to provide additional time to release of the active agent that enables the formulation to solidify without the initial loss of a substantial amount of the active agent. Among the various controlled release components disclosed, the patent discloses that the active agent may be incorporated as a conjugate with a carrier molecule, by covalently bonding the active agent to the carrier molecule, which typically will be a polymer but may be a small organic molecule such as stearic acid which is bonded through an ester or amide linkage. In Example 2 of that patent, ganirelix acetate powder is formed from a poly(sebacic acid) melt at 80 degrees Centigrade to provide a powder which is said to reduce the burst of ganirelix acetate over that observed when ganirelix acetate is simply dissolved in the polylactic acid/N-methyl-2-pyrrolidone solution.

U.S. Patent No. 5,162,057 describes coating agents for solid preparations which consist of or contain fatty acid esters of polyglycerol. The patent additionally describes that the coating agent may contain softeners such as lipids or waxes, including, inter alia, fatty acids such as stearic acid and palmitic acid, or their salts. The coating is described as being carried out in the pan coating method, or alternatively, in the form of an emulsion by melting and mixing the agent with other additives, or by heating, and then mixing with water to allow emulsification. The emulsion is sprayed on the surface of the solid preparation and dried to obtain the coated preparation.

U.S. Patent No. 4,341,759 describes a coated particle having a decreasing concentration of active agent toward the surface of the particle. The patent describes non-active lipofile substances such as waxes, fatty acids and their esters, and fatty acid alcohols, including stearic acid, glycerylmonostearate, and cetyl alcohol, for controlling the release rate. The coating is described as being applied in a coating pan or a fluidized bed apparatus.

U.S Patent No. 4,351,825 describes the manufacture of controlled release tablets in which active agent is formed in a granulated composition, then mixed with controlling agents, such as an ester of large molecule fatty acids, and compressed into tablets. The controlling agents are described as having a lipid nature and a presence in the spaces between the grains of active agent to control the penetration of water into the tablet.

Mesiha et al., "Hypoglycaemic effect of oral insulin preparations containing Brij 35, 52, 58 or 92 and stearic acid", J. Pharm. Pharmacol., 33, pgs. 733-734 (1981) describe a melt of stearic acid with the absorption promoter Brij and insulin prepared at 85 degrees Centigrade. The article speculates that micelles of emulsified stearic acid may carry insulin across the mucosal membrane and that granulations of stearic acid with Brij, being hydrophobic may enhance the stability of insulin.

Foldvari, M. and Moreland, A., in "Clinical Observations With Topical Liposome-Encapsulated Interferon Alpha For The Treatment Of Genital Papillomavirus Infections," Journal of Liposome Research, 7(1), pgs. 155-126 (1977) describe the encapsulation of alpha-interferon-2b into multilamellar liposomes composed of soya phosphatidylcholine:cholesterol:stearic acid in 2:1:1.4 molar ratio, by a solvent evaporation method.

Various salts of fatty acids and fatty acid esters are described in the prior art as useful in sustained release applications. For example, U.S. Patent No. 4,851,220 describes an oleaginous gel that may include gelling agents such as aluminum mono-fatty acid esters. U.S. Patent No. 4,650,665 describes a preferred matrix of calcium stearate, dextran and castor oil. U.S. Patent No. 5,474,980 describes compositions for the administration of polypeptides that include a biocompatible oil prepared from various fatty acid esters, e.g., triglycerides or mixtures of triglycerides and fatty acids (preferably in only minor proportions, e.g., less than about 10% free fatty acid). U.S. Patent No. 5,628,993 describes a parenteral pharmaceutical preparation formed of a matrix containing a peptide or protein and a polyglycerol diester of a saturated fatty acid, such as palmitic acid and stearic acid.

A highly effective system for controlling burst of a beneficial agent from an implant is described in related application Serial No. 08/993,208 filed December 18, 1997. Such systems are based on polymer/solvent compositions that form a gel and control the rate of ingress of water into the bulk polymeric system, thereby reducing the burst of beneficial agent which might otherwise occur upon exposure to the environment of use. Notwithstanding the effectiveness of such systems and the advantageous results achieved by controlling the bulk characteristics of the polymer matrix, it has been found that additional improvements in the controlled release of the active agent can be achieved by combining such systems with beneficial agent that is present in a controlled microenvironment within the gel as described herein.

### SUMMARY OF THE INVENTION

The invention comprises implantable compositions comprising compressed particulates of beneficial agent dispersed in a carrier and methods of manufacture. Compression reduces the ratio of surface area to mass of the particulates and reduces the rate of dissolution, dispersion or diffusion of the beneficial agent when exposed to bodily fluids in an environment of use. Practice of the present invention reduces the burst of beneficial agent, thereby minimizing potential side effects and increasing the loading capacity of the carrier for the beneficial agent so that delivery of the beneficial agent from a single implantation may be extended over a prolonged period of time. This allows for fewer implantations where administration of the beneficial agent must be carried out over an extended period of time, which may be months or even years.

In one aspect, the composition of the invention comprises a carrier, e.g., a biocompatible and bioerodible viscous gel, and particulates comprising a compressed beneficial agent, the particulates being dispersed within the carrier. The particulates may be formed of compressed beneficial agent alone, or in admixture with pharmaceutically acceptable inert ingredients. The carrier may comprise a biocompatible polymer and be combined with suitable solvents as described herein to form a gel.

In another aspect, the composition of the invention comprises a carrier, e.g., viscous gel, and particulates comprising a compressed beneficial agent, the particulates being dispersed within the carrier and the compressed particulates being formed in admixture with agents that modulate the dissolution rate of the beneficial agent when placed in the environment of use, or of compressed beneficial agent alone with a dissolution rate modulator dissolved or dispersed within the carrier, and, optionally with other pharmaceutically acceptable inert ingredients. The carrier is biocompatible and may be bioerodible.

In yet another aspect, the composition of the present invention comprises a carrier, e.g., viscous gel, and particulates comprising a compressed mixture of a beneficial active agent and an agent exhibiting a characteristic of low solubility in water, the particulates being dispersed within the carrier. The agent exhibiting the characteristic of low solubility in water may be hydrophobic. The carrier is biocompatible and may be bioerodible.

In one aspect, the hydrophobic agent may be selected from a pharmaceutically acceptable oil, fat, fatty acid, fatty acid ester, wax or derivative thereof that exhibits the hydrophobic characteristic. Preferably, the hydrophobic agent in the composition comprises a C₁₆- C₂₄ fatty acid, or an ester or pharmaceutically-acceptable salt thereof, or mixtures of the foregoing. The hydrophobic agent of the composition may comprise a mixture of stearic acid and palmitic acid. Commonly, commercial stearic acid is supplied as a mixture of stearic acid and palmitic acid, in which the stearic acid and the palmitic acid together constitute at least 90% by weight of the fatty acids of the hydrophobic agent and the stearic acid constitutes at least 40% by weight of the fatty acids of the hydrophobic agent. In a more purified form, the stearic acid and the palmitic acid together constitute at least 96% by weight of the fatty acids of the hydrophobic agent and the stearic acid constitutes at least 90% by weight of the fatty acids of the hydrophobic agent. Another commercially-available grade of stearic acid is composed of about 90% by weight of stearic acid and the remainder being mainly palmitic acid.

In another aspect of the invention, the compressed particulates of the above-described composition comprise a powder. The powder may be sized so that 90% or more of the particles pass through a sieve of 50 mesh and are retained on a sieve of 400 mesh. Often the particles are selected on the basis of passage through a 70 mesh screen and retention on a 400 mesh screen. References to mesh sizes here and throughout the description are US Standard.

The beneficial agent may be water soluble or water insoluble and may be a small molecule or a large molecule. However, the benefits of the invention may be most advantageously realized in the case of water soluble beneficial agents. Generally, the advantages of the invention will be realized in the case of water insoluble beneficial agent if the beneficial agent would otherwise interact with the components of the carrier, such as the polymer or solvent typically present in a viscous gel carrier, or with the aqueous environment of use.

The invention finds particular application to compositions in which the beneficial agent is selected from DNA, cDNA, biologically active macromolecules, proteins, peptides and polypeptides. Examples of some of such beneficial agents are human growth hormone, alpha-, beta- or gamma-interferon, erythropoietin, glugacon, calcitonin, heparin, the interleukins such as interleukin-1, interleukin-2, interleukin-11 and interleukin-12, Factor VIII, Factor IX, luteinizing hormone, relaxin, follicle-stimulating hormone, atrial natriuretic factor or filgrastim.

In another aspect of the invention, the composition comprises a polymer selected from the group consisting of polylactides, polyglycolides, polycaprolactones, polyanhydrides, polyamines, polyurethanes, polyesteramides, polyorthoesters, polydioxanones, polyacetals, polyketals, polycarbonates, polyorthocarbonates, polyphosphazenes, succinates, poly(malic acid), poly(amino acids), polyvinylpyrrolidone, polyethylene glycol, polyhydroxycellulose, chitin, chitosan, and copolymers, terpolymers and mixtures thereof.

In still another aspect, the polymer may be combined with a solvent or solvent system that restricts the bulk intake of water into the implant. Such solvents and solvent systems are identified herein, and may include alkyl or aralkyl esters of benzoic acid. In presently preferred systems the composition comprises the polymer poly(lactide-co-glycolic) acid ("PLGA") and the solvent benzyl benzoate or ethyl benzoate, in which particulates of a compressed mixture of stearic acid and the beneficial agent are dispersed.

In another aspect of the invention, the duration of release of beneficial agent may be conveniently modified by appropriate choice of the solvent for the polymer. For example, in the case of PLGA and human growth hormone, benzyl benzoate may provide a duration of release on the order of one month or longer and ethyl benzoate may provide a duration of release on the order of about one week.

In still another aspect, the invention comprises a composition comprising a bioerodible carrier comprising a polymer selected from polylactides, polyglycolides, polycaprolactones, polyanhydrides, polyamines, polyurethanes, polyesteramides, polyorthoesters, polydioxanones, polyacetals, polyketals, polycarbonates, polyorthocarbonates, polyphosphazenes, succinates, poly(malic acid), poly(amino acids), polyvinylpyrrolidone, polyethylene glycol, polyhydroxycellulose, chitin, chitosan, and copolymers, terpolymers and mixtures thereof, and a solvent selected from an alkyl or aralkyl ester of benzoic acid, and particulates comprising a compressed mixture of a beneficial agent and an agent exhibiting a characteristic of low solubility in water selected from the group consisting of a pharmaceutically acceptable oil, fat, fatty acid, fatty acid ester, wax, a derivative thereof, or a mixture of the foregoing, the particulates being dispersed within the gel.

Preferably, the hydrophobic agent in the composition comprises a C₁₆-C₂₄ fatty acid, or an ester or pharmaceutically-acceptable salt thereof, or mixtures of the foregoing. Most preferably, the hydrophobic agent of the composition comprises a mixture of stearic acid and palmitic acid. Commonly, commercial stearic acid is supplied as a mixture of stearic acid and palmitic acid, in which the stearic acid and the palmitic acid together constitute at least 90% by weight of the fatty acids of the hydrophobic agent and the stearic acid constitutes at least 40% by weight of the fatty acids of the hydrophobic agent. In a more purified form, the stearic acid and the palmitic acid together constitute at least 96% by weight of the fatty acids of the hydrophobic agent and the stearic acid constitutes at least 90% by weight of the fatty acids of the hydrophobic agent. Particulates of the above-described composition may comprise a powder. The powder may pass through a sieve of 50 mesh, and preferably 90% or more of the particulates comprising the powder pass through a 70 mesh screen and are retained on a 400 mesh screen.

In an additional aspect, the invention comprises a process for preparing the compositions of the present invention comprising the compression of granulated or powdered beneficial agent, optionally mixed with a dissolution rate modulator or an agent having a characteristic of low solubility in water to provide, after granulation, compressed particulates comprising the beneficial agent and the optional ingredients. Compression may be accomplished by compaction of the beneficial agent alone or compaction or the mixture, as the case may be, as by tableting, roller compaction, or extrusion through a suitably sized die, at pressures high enough to compact the material and produce a compacted body. The compacted body is then milled or ground to form particulates, e.g. granules or powder sized particles, of the compressed material. The compressed particulates are dispersed throughout a biocompatible carrier to form the implantable composition of the invention.

### BRIEF DESCRIPTION OF THE DRAWINGS

The foregoing and other objects, features and advantages of the present invention will be more readily understood upon reading the following detailed description in conjunction with the drawings in which:

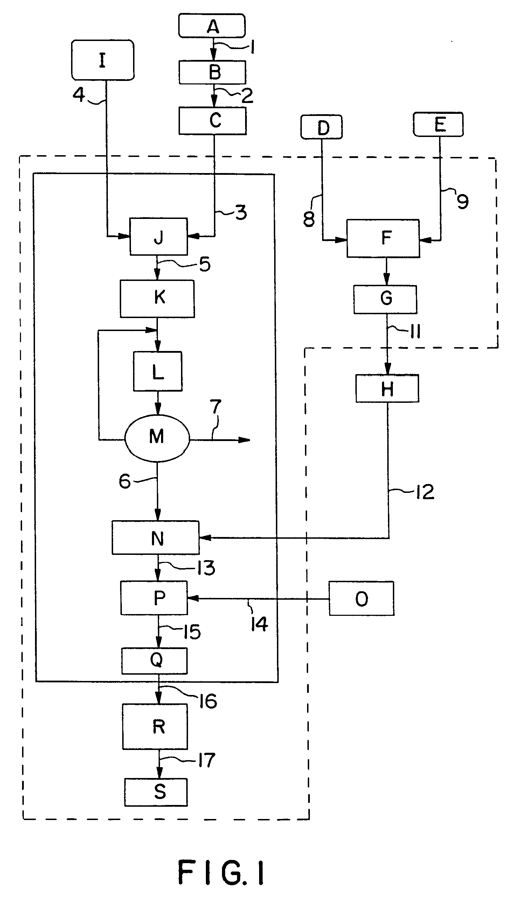

Figure 1 is a schematic flow diagram illustrating a general process for preparing compositions of the present invention;

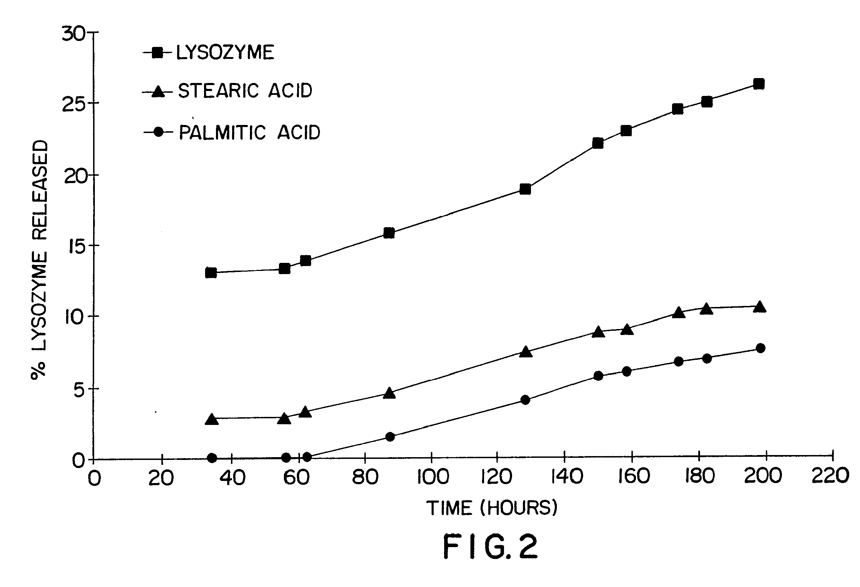

Figure 2 is a graph illustrating over a period of hours the *in vitro* release profiles of lysozyme, obtained in a USP dissolution bath of a phosphate buffer medium at 100 rpm, from three different implant compositions comprising a PLGA polymer gel, in which, respectively, lysozyme is alone present in the polymer gel (square symbols), lysozyme is present as a compressed mixture with stearic acid (triangle symbols), and lysozyme is present as a compressed mixture with palmitic acid (circle symbols);

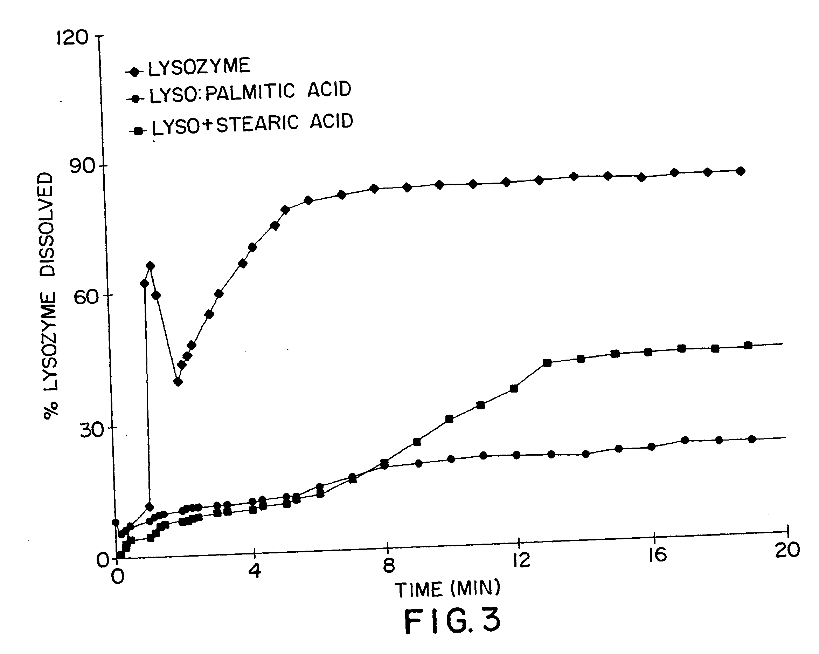

Figure 3 is a graph illustrating over a period of minutes the *in vitro* release profiles of lysozyme, obtained in a USP dissolution bath of a phosphate buffer medium at 100 rpm, from three different implant compositions comprising a PLGA polymer gel, in which, respectively, lysozyme is alone present in the polymer gel (diamond symbols), lysozyme is present as a compressed mixture with stearic acid (square symbols), and lysozyme is present as a compressed mixture with palmitic acid (circle symbols);

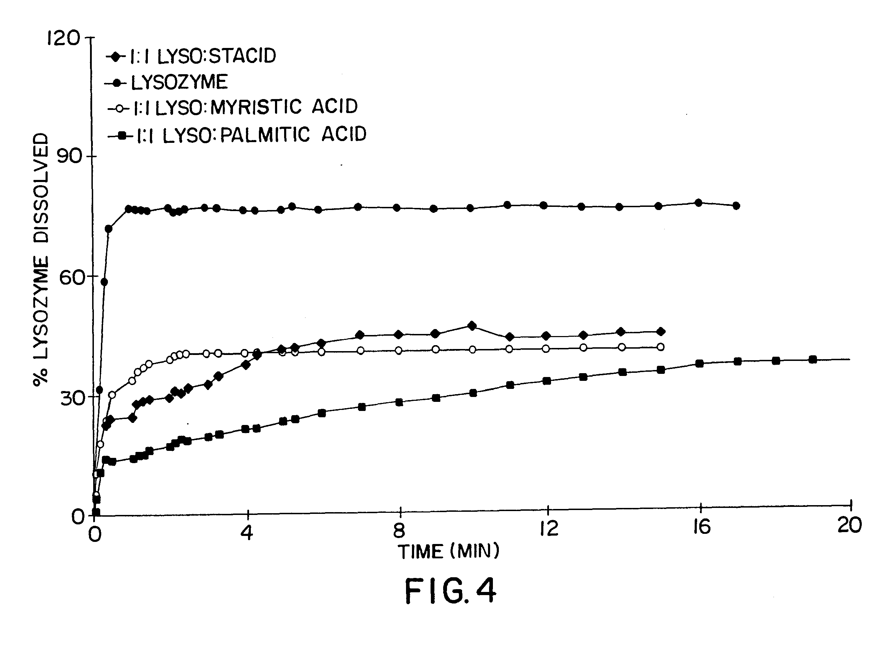

Figure 4 is a graph illustrating over a period of minutes the *in vitro* release profiles of lysozyme, obtained in a USP dissolution bath of a phosphate buffer medium at 100 rpm, from three different implant compositions comprising a PLGA polymer gel, in which, respectively, lysozyme is alone present in the polymer gel (filled circle symbols, top curve), lysozyme is present as a compressed mixture in a 1:1 ratio with myristic acid (partially filled circles), lysozyme is present as a compressed mixture in a 1:1 ratio with stearic acid (diamond symbols) and lysozyme is present as a compressed mixture in a 1:1 ratio with palmitic acid (square symbols);

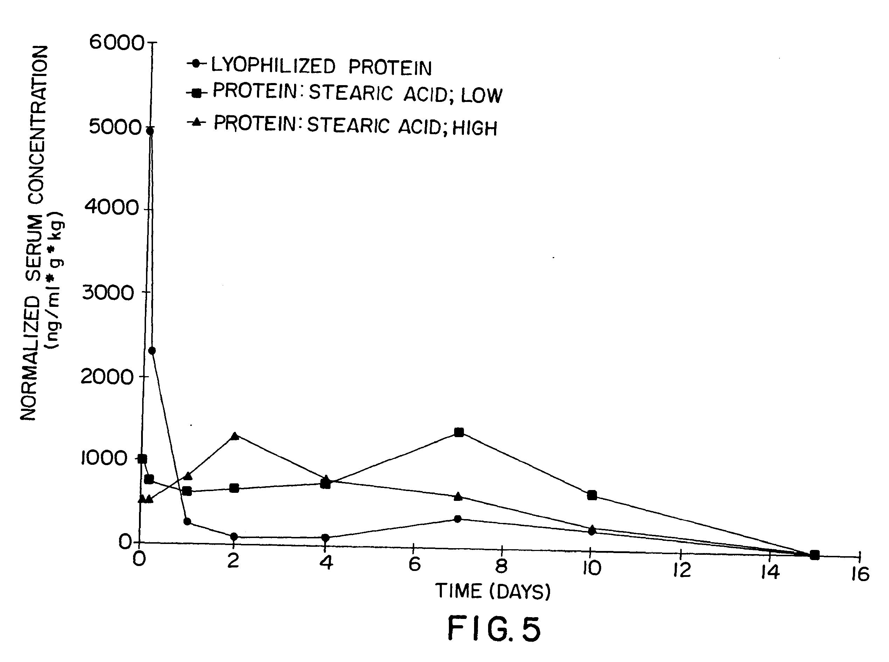

Figure 5 is a graph illustrating the *in vivo* release from a representative injectable depot as measured in rat serum of two different human growth hormone ("hGH") /stearic acid formulations (1:1 hGH:stearic acid, square symbols) and 1:2 hGH:stearic acid, triangle symbols) compared to the release of hGH particles alone (circle symbols); and

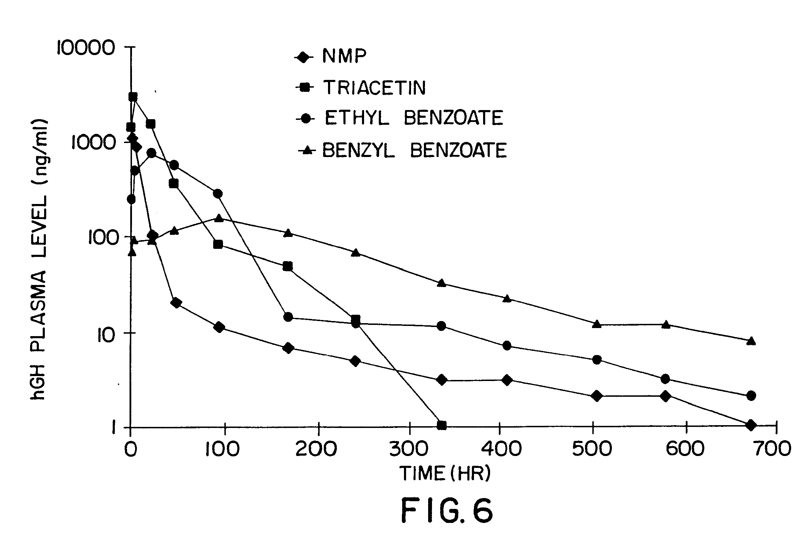

Figure 6 is a graph illustrating the *in vivo* release as measured in rat serum of human growth hormone particles, which are formed as compressed particulates with stearic acid in accordance with the description herein, from a PLGA gel containing 2-N-methylpyrrolidone (diamond symbols), triacetin (square symbols), ethyl benzoate (circle symbols) and benzyl benzoate (triangle symbols), respectively.

### DETAILED DESCRIPTION OF THE INVENTION

The present invention is directed to improved compositions useful for systemically or locally administering a beneficial agent to a subject by implanting in the subject an implantable system comprising the compositions of the invention and methods for manufacturing such compositions.

### Definitions

The term "AUC" means the area under the curve obtained from an *in vivo* assay in a subject by plotting blood plasma concentration of the beneficial agent in the subject against time, as measured from the time of implantation of the composition, to a time *"t"* after implantation. The time t will correspond to the delivery period of beneficial agent to a subject.

The term "beneficial agent" means an agent that effects a desired beneficial, often pharmacological, effect upon administration to a human or an animal, whether alone or in combination with other pharmaceutical excipients or inert ingredients.

The term "burst index" means, with respect to a particular composition intended for systemic delivery of a beneficial agent, the quotient formed by dividing (i) the AUC calculated for a predetermined time period after implantation of the composition into a subject divided by the number of hours in the predetermined period, by (ii) the AUC calculated for the time period of delivery of beneficial agent, divided by the number of hours in the total duration of the delivery period. For purposes of reference to numeric values of burst indices referred to herein, the predetermined period will be 24 hours. However, it is recognized that in other applications the duration of the predetermined period may depend on the nature of the beneficial agent and the therapeutic application, such that the predetermined period may be a short, but measurable, period immediately after implantation or a longer period. However, in most applications the longer period would not be expected to extend past 96 hours.

The term "compressed" means, with respect to a material or a mixture of materials, that the material or mixture of materials is compressed or compacted such that its bulk density after compression or compaction is greater than it was prior to compression or compaction. Compression or compaction is conveniently effected by tableting or pelletizing of the aforementioned mixture using conventional processes or by roller compaction or extrusion of the aforementioned materials using conventional processes.

The term "compressed particulates" means, with respect to the beneficial agent, or a mixture of the beneficial agent and a rate dissolution modulator, or a mixture of beneficial agent and agent exhibiting a characteristic of low solubility in water, that the particulates are formed from compressed or compacted particles of beneficial agent, or a compressed or compacted mixture of particles of beneficial agent and a dissolution rate modulator, or a compressed or compacted mixture of the beneficial agent and agent exhibiting a characteristic of low solubility in water, respectively. The compressed particulates may be formed by granulation from a larger compressed or compacted body, such as formed by a tabletting, pelletizing, roller compacting or extrusion operation, by crushing the body to form particulates, which may be granules or powder. For purposes hereof, particulates generally have a maximum dimension or size of between about 0.1 micron to about 500 microns, more often 5 microns to about 400 microns. "Granules" generally will refer to particulates having an average size greater than that of powder. The term "particulates" is inclusive of granules and powder.

The phrase " dispersed" is intended to encompass all means of establishing a presence of compressed particulates of the beneficial agent, or a mixture of the beneficial agent and a dissolution rate modulator, or a mixture of beneficial agent and agent exhibiting a characteristic of low solubility in water, in the carrier, and includes dispersion, suspension and the like.

The term "systemic " means, with respect to delivery or administration of a beneficial agent to a subject, that beneficial agent is detectable at a biologically-significant level in the blood plasma of the subject.

The term "local " means, with respect to delivery or administration of a beneficial agent to a subject, that beneficial agent is delivered to a localized site in the subject but is not detectable at a biologically-significant level in the blood plasma of the subject.

The term "gel" or "gel vehicle", which may be used interchangeably herein, means the composition formed by mixture of a polymer and solvent in the absence of the beneficial agent, and encompasses, for example, polymer solutions, hydrogels, emulsions, gelatins, and the like.

The term "prolonged period" means a period of time over which release of a beneficial agent from the implant of the invention occurs, which will generally be about one week or longer, and preferably about 30 days or longer, but may be 3 months or longer.

The term "initial burst" means, with respect to a particular composition of this invention, the quotient obtained by dividing (i) the amount by weight of beneficial agent released from the composition in a predetermined initial period of time after implantation, typically a time period just after implantation to a time period of up to 96 hours, by (ii) the total amount of beneficial agent that is to be delivered from an implanted composition. It is understood that the initial burst may vary depending on the shape and surface area of the implant. Accordingly, the percentages and burst indices associated with initial burst described herein are intended to apply to compositions tested in a form resulting from dispensing of the composition from a standard syringe.

The term "stearic acid" as used herein, unless the context requires otherwise, refers to commercially available mixtures of stearic acid (C₁₈H₃₆O₂) and palmitic acid (C₁₆H₃₂O₂) that are sold as stearic acid. Preferably, the content of the stearic acid in the mixture is not less than 40% and the sum of the two acids is not less than 90% of the mixture. Stearic acid is typically manufactured by hydrogenation of cottonseed and other vegetable oils or by hydrolysis of fat under high pressure and high temperature, yielding the aforementioned mixture.

The term "subject" means, with respect to the administration of a composition of the invention, an animal or a human being.

Since all solvents, at least on a molecular level, will be soluble in water (i.e., miscible with water) to some very limited extent, the term "immiscible" as used herein means that 7% or less by weight of the solvent is soluble in or miscible with water. For the purposes of this disclosure, solubility values of solvent in water are considered to be determined at 20°C. Since it is generally recognized that solubility values as reported may not always be conducted at the same conditions, solubility limits recited herein as percent by weight miscible or soluble with water as part of a range or upper limit may not be absolute. For example, if the upper limit on solvent solubility in water is recited herein as "7% by weight", and no further limitations on the solvent are provided, the solvent "triacetin", which has a reported solubility in water of 7.17 grams in 100 ml of water, is considered to be included within the limit of 7%. A solubility limit in water of less than 7% by weight as used herein does not include the solvent triacetin or solvents having solubilities in water equal to or greater than triacetin.

The present invention comprises a bioerodible and biocompatible carrier, e.g., viscous gel, and particulates comprising a compressed beneficial agent, the particulates being dispersed within the carrier. The particulates may be formed of compressed beneficial agent alone, or in admixture with pharmaceutically acceptable inert ingredients. Additionally, prior to compression the beneficial agent may be mixed with a dissolution rate modulator, or an agent having low solubility in water, such as a hydrophobic agent. The bioerodible carrier may comprise a polymer as described herein, and be combined with suitable solvents as described herein to form a viscous gel, such as those gels that limit bulk water uptake.

Compression of the beneficial agent into tablets and subsequent grinding affords particulates of beneficial agent in which the surface area to mass ratio is less than in the case where particulates of beneficial agent are formed by conventional methods, such as spray drying, precipitation from solution, and the like. While the reduction in the ratio of surface area to mass may not significantly decrease the rate of water uptake, and subsequent dissolution or dispersion of the beneficial agent, in in vitro dissolution studies, the combination of particulates so compressed in the viscous polymer gels as described herein, provides significant reduction in water uptake by the particulates as compared to non-compressed particles in such gels.

For example, while non-compressed particulates of hGH formed by spray drying with average particle diameters on the order of 5 microns may dissolve in a USP dissolution assay in a time period on the order of seconds, compressed particles of the same or similar size range may dissolve on the order of minutes. In viscous polymer gels formed with immiscible solvents as described herein, compressed hGH particles may retain their integrity and continue the process of dissolution and diffusion from the implant over a period of days or weeks. Reduced water uptake in the microenvironment of the particulates of beneficial agent modulates or eliminates burst and enables prolonged release of beneficial agent from the implant.

For description purposes, the manufacture of the compositions of the invention will be illustrated with mixtures of beneficial agent and one or more agents exhibiting low solubility in water, such as hydrophobic agents. Compressed particulates of beneficial agent alone, which may contain pharmaceutically acceptable excipient, or optionally mixed with a dissolution rate modulator, may be formed in the same manner as with the hydrophobic agents, except that the steps involving hydrophobic agents are eliminated in the case of beneficial agent alone. If the particulates comprise a mixture of a beneficial agent and a dissolution rate modulator, substitution of the rate modulator for the hydrophobic agent in the described process generally will provide the requisite material for further processing. Typically, then, compressed tablets of beneficial agent, either alone or in admixture, are formed by conventional tableting methods, the tablets are ground or milled and the resulting particulates are sized through sieving screens to provide particulates in size ranges as described elsewhere herein. After sizing, the particulates are combined with the gel, and in a preferred embodiment, loaded into syringes. Alternatively, the beneficial agent either alone or in the mixtures described above can be compacted with a roller compactor and ten ground or milled to the appropriately sized particulates.

Accordingly, in a one embodiment of the invention, compressed particulates comprising a compressed mixture of beneficial agent and an agent exhibiting a characteristic of low solubility in water are dispersed in an implantable carrier. The compressed particulates are conveniently formed by initially tableting or pelletizing a mixture of beneficial agent and the agent exhibiting a characteristic of low solubility in water. While not an absolute requirement, preferably the two components will be intimately mixed to substantial homogeneity such that the concentration of the various components is largely the same throughout the mixture. In order to accomplish the desired degree of mixing, the beneficial agent and agent exhibiting a characteristic of low solubility in water may be ground to the powdered state, if not already in such a state, prior to their being mixed.

After mixing, the particulate mixture is compressed to form a tablet or pellet or roller compacted or extruded to form a compressed body that has a density greater than that of the aggregation of particles of the mixture prior to the compression step. Conveniently, the mixture of beneficial agent and agent exhibiting a characteristic of low solubility in water is tableted in a conventional tabletting press such as those well known in the pharmaceutical manufacturing industry. For low volume production, a simple, manual Carver press may be employed. For greater production volume, automated presses may be employed. A number of commercially available tabletting presses are described in Remington's Pharmaceutical Sciences, Eighteenth Edition, pages 1647-1653 (1990), Mack Publishing Company, Easton, Pennsylvania, and includes presses such as those manufactured by Stokes-Pennwalt, Manesty and others. Subsequently, the tableted mixture is crushed or milled to form compressed particulates of the mixture, which can be sieved through sized screens to provide compressed particulates in a desired particle size range. As described elsewhere, roller compactors and extruders may be utilized also to form compacted articles, which may be ground or milled and sized to obtain the particulates for dispersal throughout the carrier. Commercial compactors are available from Alexander Werk, Remsheid, Germany and Gerteis, Jona, Switzerland.

In the case of beneficial agents that may be sensitive to heat, such as proteins or peptides that may be prone to denaturing under sustained elevated temperature conditions, the time of compression may be kept relatively short. Consequently, any rise in temperature of the compressed composition during the tableting of the composition is limited to a short duration of time. Also, the die and punch of the press provides a convenient heat sink for the dissipation of heat that might otherwise detrimentally affect the beneficial agent. Even if there is a rise in temperature it will be transient and not adversely affect the beneficial agent, e.g., protein, peptide or other substance that may be heat sensitive.

The compressed particulates are then dispersed throughout a carrier, such as a biocompatible polymer, that may be bioerodible. The carrier may be solid or semi-solid that is implanted in the subject surgically, or the carrier may be prepared for implantation by injection as a liquid which will solidify in-situ or as a gel. For purposes of maintaining the dispersion of particulates throughout the carrier, in the case of implantation by injection the use of a viscous gel is preferred.

Agents that exhibit a characteristic of low solubility in water and are useful in the present invention may include anionic, cationic, amphoteric and nonionic surfactants that have a solubility in water that is less than the solubility in water of the beneficial agent and other hydrophobic materials that do not interact detrimentally with the beneficial agent and are compatible with compression when in admixture with the beneficial agent. Suitable agents may be selected from surfactants such as those described in Remington's Pharmaceutical Sciences, supra, at pages 267-268. Presently preferred agents include C₁₆ - C₂₄ long chain fatty acids, esters of such long chain fatty acids, pharmaceutically acceptable salts and mixtures thereof. Especially preferred are stearic acid, palmitic acid, and myristic acid, esters and pharmaceutically salts thereof, and mixtures of the foregoing. Other agents that impart hydrophobicity to the compressed particulates containing the beneficial agent may include collagen, waxes, lipids, liposomes and polymeric materials.

Dissolution rate modulators may be substituted for the agents exhibiting a characteristic of low solubility in water in the particulate mixture, and the selection of such modulator(s) may depend on the physiochemical characteristics of the beneficial agent. In some circumstances, it may be desirable to distribute a dissolution rate modulator throughout the bioerodible carrier in which the compressed particulates are dispersed.

Dissolution rate modulators have been described in the related application and published patents and literature, and include, for example, metal cations such as described in US Patent 5,656,297 and agents described in US Patent 5,674,534, which are incorporated herein by reference. Additionally, dissolution rate modulators may include materials that create a volume exclusion effect and/or scavenge water in the microenvironment of the particulates. To the extent such materials attract water, it is important to select those that have a net scavenging effect so that more water is not drawn to the microenvironment of the particulates than would be present in the absence of the scavenging material. This typically, can be determined by assessing the total uptake of water of a mixture of the gel vehicle and the beneficial agent particulates with or without the scavenging material. Such modulators may be selected from mono-, di-, tricarboxylic acids, esters, salts and alcohols formed therefrom, water soluble polymers such as polyethylene glycol and poloxamers. Polyethylene glycols having a molecular weight of between 3,000 -10,000 daltons may be used, but generally the higher molecular weight materials are preferable. Such modulators may be combined with the beneficial agent by conventional methods, e.g., spray drying, lyopholization or pan coating, prior to the compaction step.

The beneficial agent can be any physiologically or pharmacologically active substance or substances optionally in combination with pharmaceutically acceptable carriers and additional ingredients such as antioxidants, stabilizing agents, permeation enhancers, etc. that do not substantially adversely affect the advantageous results that can be attained by the present invention. The beneficial agent may be any of the agents which are known to be delivered to the body of a human or an animal and that are preferentially soluble in water rather than in the polymer-dissolving solvent. These agents include drug agents, medicaments, vitamins, nutrients, or the like. Included among the types of agents which meet this description are lower molecular weight compounds, biologically active macromolecules, proteins, peptides, genetic material, nutrients, vitamins, food supplements, sex sterilants, fertility inhibitors and fertility promoters.

Drug agents which may be delivered by the present invention include drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Suitable agents may be selected from, for example, DNA, cDNA, proteins, enzymes, hormones, polynucleotides, nucleoproteins, polysaccharides, glycoproteins, lipoproteins, polypeptides, steroids, analgesics, local anesthetics, antibiotic agents, anti-inflammatory corticosteroids, ocular drugs and synthetic analogs of these species.

Examples of drugs which may be delivered by the composition of the present invention include, but are not limited to, prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, digoxin, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17α-hydroxyprogesterone acetate, 19-nor-progesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, milrinone, mandol, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat, captopril, ramipril, famotidine, nizatidine, sucralfate, etintidine, tetratolol, minoxidil, chlordiazepoxide, diazepam, amitriptyline, and imipramine.

Additional, examples are proteins and peptides which include, but are not limited to, bone morphogenic proteins, insulin, colchicine, glucagon, thyroid stimulating hormone, parathyroid and pituitary hormones, calcitonin, renin, prolactin, corticotrophin, thyrotropic hormone, follicle stimulating hormone, chorionic gonadotropin, gonadotropin releasing hormone, bovine somatotropin, porcine somatotropin, oxytocin, vasopressin, GRF, somatostatin, lypressin, pancreozymin, luteinizing hormone, LHRH, LHRH agonists and antagonists, leuprolide, interferons such as interferon alpha-2a, interferon alpha-2b, and consensus interferon, interleukins, growth hormones such as human growth hormone and its derivatives such as methione-human growth hormone and des-phenylalanine human growth hormone, bovine growth hormone and porcine growth hormone, fertility inhibitors such as the prostaglandins, fertility promoters, growth factors such as insulin-like growth factor, coagulation factors, human pancreas hormone releasing factor, analogs and derivatives of these compounds, and pharmaceutically acceptable salts of these compounds, or their analogs or derivatives.

The present invention has particular application to the delivery of beneficial agents selected from DNA, cDNA, biologically active macromolecules, proteins, peptides and polypeptides. Examples of some of such beneficial agents are human growth hormone, alpha-, beta- or gamma-interferon, erythropoietin, glugacon, calcitonin, heparin, the interleukins such as interleukin-1, interleukin-2, interleukin-11 and interleukin-12, Factor VIII, Factor IX, luteinizing hormone, relaxin, follicle-stimulating hormone, atrial natriuretic factor or filgrastim.

The present invention also finds application with chemotherapeutic agents for the local application of such agents to avoid or minimize systemic side effects. Gels of the present invention containing chemotherapeutic agents may be injected directly into the tumor tissue for sustained delivery of the chemotherapeutic agent over time. In some cases, particularly after resection of the tumor, the gel may be implanted directly into the resulting cavity or may be applied to the remaining tissue as a coating. In cases in which the gel is implanted after surgery, it is possible to utilize gels having higher viscosities since they do not have to pass through a small diameter needle. Representative chemotherapeutic agents that may be delivered in accordance with the practice of the present invention include, for example, carboplatin, cisplatin, paclitaxel, BCNU, vincristine, camptothecin, etopside, cytokines, ribozymes, interferons, oligonucleotides and oligonucleotide sequences that inhibit translation or transcription of tumor genes, functional derivatives of the foregoing, and generally known chemotherapeutic agents such as those described in U.S. Patent 5,651,986. The present application has particular utility in the sustained delivery of water soluble chemotherapeutic agents, such as for example cisplatin and carboplatin and the water soluble derivatives of paclitaxel. Those characteristics of the invention that minimize the burst effect are particularly advantageous in the administration of water soluble beneficial agents of all kinds, but particularly those compounds that are clinically useful and effective but may have adverse side effects.

To the extent not mentioned above, the beneficial agents described in aforementioned U.S. Patent No. 5,242,910 can also be used. One particular advantage of the present invention is that materials, such as proteins, as exemplified by the enzyme lysozyme, and cDNA, and DNA incorporated into vectors both viral and nonviral, which are difficult to microencapsulate or process into microspheres can be incorporated into the compositions of the present invention without the level of degradation caused by exposure to high temperatures and denaturing solvents often present in other processing techniques.

The beneficial agent may be obtained as a powder or, if a liquid, it may be incorporated into a porous solid particle, such as anhydrous calcium phosphate that is sold under the trademark Fujicalin by Fuji Chemical Industries (U.S.A.) Inc., Engelwood, New Jersey, or powdered magnesium aluminometasilicate and sold under the trademark Neusilin by Fuji Chemical Industry Co., Ltd., Toyam, Japan.

The beneficial agent particles suitable for compacting typically have an average particle size of from about 0.1 to about 200 microns, preferably from about 1 to about 100 microns and often from 1 to 50 microns, and most preferably 2-10 microns. Conventional lyophilization processes can also be utilized to form particles of beneficial agents of varying sizes using appropriate freezing and drying cycles.

The implantable carrier for the beneficial agent may be formed as a gel. The gel may be viscous and formed of a polymer. The gel may be formed of components such that bulk water uptake in the implant also is restricted. Preferred carrier system include those systems that have been described in detail in copending application Serial No. 08/993,208 filed December 18, 1997 and its corresponding PCT counterpart application bearing international publication number WO 98/26359 and international publication date July 2, 1998. That published application may be referred to for details of bulk polymer systems that are particularly useful with the present invention. However, other polymer systems may be used as well.

The polymer, solvent and other agents of the invention should be biocompatible; that is they should not cause undue irritation or necrosis in the environment of use. The environment of use is a fluid environment and may comprise a subcutaneous or intramuscular portion or body cavity of a human or animal.

Polymers that may be useful in the invention may be biodegradable and may include, but are not limited to polylactides, polyglycolides, polycaprolactones, polyanhydrides, polyamines, polyurethanes, polyesteramides, polyorthoesters, polydioxanones, polyacetals, polyketals, polycarbonates, polyorthocarbonates, polyphosphazenes, succinates, poly(malic acid), poly(amino acids), polyvinylpyrrolidone, polyethylene glycol, polyhydroxycellulose, chitin, chitosan, and copolymers, terpolymers and mixtures thereof.

Presently preferred polymers are polylactides, that is, a lactic acid-based polymer that can be based solely on lactic acid or can be a copolymer based on lactic acid and glycolic acid which may include small amounts of other comonomers that do not substantially affect the advantageous results which can be achieved in accordance with the present invention. As used herein, the term "lactic acid" includes the isomers L-lactic acid, D-lactic acid, DL-lactic acid and lactide while the term "glycolic acid" includes glycolide. Most preferred are poly(lactide-co-glycolide)copolymers, commonly referred to as PLGA. The polymer may have a monomer ratio of lactic acid/glycolic acid of from about 100:0 to about 15:85, preferably from about 60:40 to about 75:25 and an especially useful copolymer has a monomer ratio of lactic acid/glycolic acid of about 50:50.

The lactic acid-based polymer has a number average molecular weight of from about 1,000 to about 120,000, preferably from about 5,000 to about 30,000 as determined by gas phase chromatography. As indicated in aforementioned U.S. Patent No. 5,242,910, the polymer can be prepared in accordance with the teachings of U.S. Patent No. 4,443,340. Alternatively, the lactic acid-based polymer can be prepared directly from lactic acid or a mixture of lactic acid and glycolic acid (with or without a further comonomer) in accordance with the techniques set forth in U.S. Patent No. 5,310,865. The contents of all of these patents are incorporated by reference.

Suitable lactic acid-based polymers are available commercially. For instance, 50:50 lactic acid:glycolic acid copolymers having molecular weights of 5,000, 10,000, 30,000 and 100,000, preferably about 8,000 to 13,000, and most preferably about 10,000, and a wide variety of end groups to alter susceptibility to hydrolysis and subsequent breakdown of the polymer chain are available from Boehringer Ingelheim (Petersburg, VA). Additional polymers include, for example, Poly (D,L-lactide-co-glycolide) 50:50 RESOMER® L104, PLGA-L104, code no. 33007, Poly (D,L-lactide-co-glycolide) 50:50 RESOMER® RG206, PLGA-206, code no. 8815, Poly (D,L-lactide-co-glycolide) 50:50 RESOMER® RG502, PLGA-502, code 0000366, Poly (D,L-lactide-co-glycolide) 50:50 RESOMER® RG502H, PLGA-502H, code no. 260187, Poly (D,L-lactide-co-glycolide) 50:50 RESOMER® RG503, PLGA-503, code no. 0080765, Poly (D,L-lactide-co-glycolide) 50:50 RESOMER® RG506, PLGA-506, code no. 95051, Poly (D,L-lactide-co-glycolide) 50:50 RESOMER® RG755, PLGA-755, code no. 95037, (Boehringer Ingelheim Chemicals, Inc., Petersburg, VA)

The biocompatible polymer is present in the gel composition in an amount ranging from about 5 to about 80% by weight, preferably from about 30 to about 70% by weight and often 40 to 60% by weight of the viscous gel, the viscous gel comprising the combined amounts of the biocompatible polymer and the solvent. The solvent will be added to polymer in amounts described herein, to provide implantable or injectable viscous gels.

The solvent should be biocompatible, and preferably should form a viscous gel with the polymer, and restrict water uptake into the implant. The solvent may be a single solvent or a mixture of solvents exhibiting the foregoing properties. The term "solvent", unless specifically indicated otherwise, means a single solvent or a mixture of solvents. A broad range of solvents may be utilized in the present invention. Water soluble solvents, including those that are highly, moderately or barely soluble, as well as solvents that have such limited solubility so as to be considered insoluble or immiscible in water may be used. Because the present invention establishes a microenvironment about the beneficial agent that tends to retard the uptake of water in the vicinity of and by the beneficial agent, solvents for the polymer that are soluble in water may be employed, even though presently such solvents may not be preferred. Such solvents may include for example, but are not limited, to triacetin, diacetin, tributyrin, esters of citric acid such as triethyl citrate, tributyl citrate, acetyl triethyl citrate, and acetyl tributyl citrate, triethylglycerides, triethyl phosphate, diethyl phthalate, diethyl tartrate, mineral oil, polybutene, silicone fluid, glylcerin, ethylene glycol, polyethylene glycol, octanol, ethyl lactate, propylene glycol, propylene carbonate, ethylene carbonate, butyrolactone, ethylene oxide, propylene oxide, N-methyl-2-pyrrolidone, 2-pyrrolidone, glycerol formal, methyl acetate, ethyl acetate, methyl ethyl ketone, dimethylformamide, dimethyl sulfoxide, tetrahydrofuran, caprolactam, decylmethylsulfoxide, oleic acid, and 1-dodecylazacyclo-heptan-2-one, and mixtures thereof.

It is presently preferable to control the bulk water uptake by the implant by use of solvents that substantially restrict the uptake of water by the implant. Such solvents may be characterized as immiscible in water, i.e., having a solubility in water of less than 7% by weight. Preferably, the solvents are five weight percent or less soluble in water; more preferably three weight percent or less soluble in water; and even more preferably one weight percent or less soluble in water. Most preferably the solubility of the solvent in water is equal to or less than 0.5 weight percent.

Solvents having the above solubility parameters may be selected from the lower alkyl and aralkyl esters of aryl acids such as benzoic acid, the phthalic acids, salicylic acid, lower alkyl esters of citric acid, such as triethyl citrate and tributyl citrate and the like, and aryl, aralkyl and lower alkyl ketones. Among preferred solvents are those having solubilities within the foregoing range selected from (i) compounds having the following structural formulas : 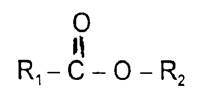 and (i) 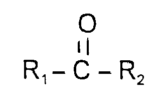 in which R₁ is aryl or aralkyl, R₂ is lower alkyl or aralkyl, and R₁ and R₂ are optionally the same or different, with the proviso that when each of R₁ and R₂ are lower alkyl, the total carbon atoms in R₁ and R₂ combined are 4 or more, and (ii) lower alkyl and aralkyl esters of phthalic acid, isophthalic acid and terephtalic acid and (iii) lower alkyl and aralkyl esters of citric acid. For the purposes hereof, lower alkyl means straight or branched chain hydrocarbons having 1-6 carbon atoms, optionally substituted with non-interfering substituents; aralkyl means (lower alkyl)phenyl, e.g., benzyl, phenethyl, 1-phenylpropyl, 2-phenylpropyl, and the like wherein the alkyl moiety contains from 1-6 carbon atoms; and aryl means phenyl, optionally substituted by non-interfering substituents. Many of the solvents useful in the invention are available commercially (Aldrich Chemicals, Sigma Chemicals) or may be prepared by conventional esterification of the respective arylalkanoic acids using acid halides, and optionally esterification catalysts, such as described in US Patent No. 5,556,905, which is incorporated herein by reference, and in the case of ketones, oxidation of their respective secondary alcohol precursors.

Art recognized benzoic acid derivatives from which solvents having the requisite solubility may be selected include: 1,4-cyclohexane dimethanol dibenzoate, diethylene glycol dibenzoate, dipropylene glycol dibenzoate, polypropylene glycol dibenzoate, propylene glycol dibenzoate, diethylene glycol benzoate and dipropylene glycol benzoate blend, polyethylene glycol (200) dibenzoate, iso decyl benzoate, neopentyl glycol dibenzoate, glyceryl tribenzoate, pentaerylthritol tetrabenzoate, cumylphenyl benzoate, trimethyl pentanediol dibenzoate.

Art recognized phthalic acid derivatives from which solvents having the requisite solubility may be selected include: Alkyl benzyl phthalate, bis-cumylphenyl isophthalate, dibutoxyethyl phthalate, dimethyl phthalate, dimethyl phthalate, diethyl phthalate, dibutyl phthalate, diisobutyl phthalate, butyl octyl phthalate, diisoheptyl phthalate, butyl octyl phthalate, diisonoyl phthalate, nonyl undecyl phthalate, dioctyl phthalate, di-iso octyl phthalate, dicapryl phthalate, mixed alcohol phthalate, di-(2-ethylhexyl) phthalate, linear heptyl, nonyl, phthalate, linear heptyl, nonyl, undecyl phthalate, linear nonyl phthalate, linear nonyl undecyl phthalate, linear dinoyl, didecyl phthalate (diisodecyl phthalate), diundecyl phthalate, ditridecyl phthalate, undecyldodecyl phthalate, decyltridecyl phthalate, blend (50/50) of dioctyl and didecyl phthalates, butyl benzyl phthalate, and dicyclohexyl phthalate.

Preferred solvents include the lower alkyl and aralkyl esters of the aryl acids described above. Representative acids are benzoic acid and the phthalic acids, such as phthalic acid, isophthalic acid, and terephathalic acid. Most preferred solvents are derivatives of benzoic acid and include, but are not limited to, methyl benzoate, ethyl benzoate, n-propyl benzoate, isopropyl benzoate, butyl benzoate, isobutyl benzoate, sec-butyl benzoate, *tert*-butyl benzoate, isoamyl benzoate and benzyl benzoate, with benzyl benzoate being most especially preferred. Preferred solvent mixtures are those in which benzyl benzoate is the primary solvent, and mixtures formed of benzyl benzoate and either triacetin, tributyl citrate, triethyl citrate or N-methyl-2-pyrrolidone. Preferred mixtures are those in which benzyl benzoate is present by weight in an amount of 50% or more, more preferably 60% or more and most preferably 80% or more of the total amount of solvent present. Especially preferred mixtures are those of 80/20 mixtures by weight of benzyl benzoate/triacetin and benzyl benzoate/N-methyl-2-pyrrolidone.

Additional solvents may include diethyl tartrate, diethyl maleate, methyl salicylate, p-anisaldehyde, phenyl acetate, benzy salicylate, benzyl acetate, methyl phenyl acetate, anisole, and diethyl malonate.

It has been found that the solvents described above having a miscibility in water of less than 7% by weight may be mixed with one or more additional miscible solvents ("component solvents"). Component solvents compatible and miscible with the primary solvent may have a higher miscibility with water and the resulting mixtures may still exhibit significant restriction of water uptake into the implant. Such mixtures will be referred to as "component solvent mixtures." Useful component solvent mixtures may exhibit solubilities in water greater than the primary solvents themselves, typically between 0.1 weight percent and up to and including 50 weight percent, preferably up to and including 30 weight percent, and most preferably up to an including 10 weight percent, without detrimentally affecting the restriction of water uptake exhibited by the implants of the invention. Especially preferred are component solvent mixtures having a solubility in water of about 0.1% to about 7% by weight.

Component solvents useful in component solvent mixtures are those solvents that are miscible with the primary solvent or solvent mixture, and include, but are not limited, to triacetin, diacetin, tributyrin, triethyl citrate, tributyl citrate, acetyl triethyl citrate, acetyl tributyl citrate, triethylglycerides, triethyl phosphate, diethyl phthalate, diethyl tartrate, mineral oil, polybutene, silicone fluid, glylcerin, ethylene glycol, polyethylene glycol, octanol, ethyl lactate, propylene glycol, propylene carbonate, ethylene carbonate, butyrolactone, ethylene oxide, propylene oxide, N-methyl-2-pyrrolidone, 2-pyrrolidone, glycerol formal, methyl acetate, ethyl acetate, methyl ethyl ketone, dimethylformamide, dimethyl sulfoxide, tetrahydrofuran, caprolactam, decylmethylsulfoxide, oleic acid, and 1-dodecylazacyclo-heptan-2-one, and mixtures thereof.

In an especially preferred embodiment, the solvent is selected from lower alkyl and aralkyl esters of benzoic acid and the polymer is a lactic-acid based polymer, most preferably PLGA, having a number average molecular weight of between about 8,000 to about 13,000, preferably about 10,000. Presently, the most preferred solvents are benzyl benzoate and the lower alkyl esters of benzoic acid, particularly ethyl benzoate. PLGA/benzyl benzoate-based gels exhibit delivery periods, on the order of one month or greater duration. Delivery periods on the order of one week are observed for PLGA/ethyl benzoate-based gels, the benzyl benzoate and ethyl benzoate gels having substantially the same compositions except for the difference in solvent. Variation in the delivery period is a useful tool for the medical practitioner. For example, PLGA/ethyl benzoate/human growth hormone ("hGH") gels prepared in accordance with the procedures described herein provide approximately one week of delivery of hGH. That delivery pattern may be of benefit in the treatment of pediatric patients where close monitoring of patient growth is desirable and the administration of hGH can be stopped or started as necessary, yet without the inconvenience of daily injections. The benzoic acid esters may be used alone or in a mixture with other miscible solvents, e.g., triacetin, as described herein.

Implants are preferably prepared as viscous gels in which the compressed particulates of the beneficial agent, the mixture of the beneficial agent and the dissolution rate modulator, or the mixture of beneficial agent and agent exhibiting a characteristic of low solubility in water, are dispersed substantially throughout, and such compositions are useful both for systemic and local administration of beneficial agent, whether or not initial burst is an important consideration. Typically, the compressed particulates will be loaded into the gel vehicle at 0.1-50% by weight, preferably 1-20% by weight. Additionally, use of esters of benzoic acid provides increased control of water migration resulting in increased stability of beneficial agent. The low water uptake, i.e., limited water migration into the gel composition after implantation, permits the practitioner of the invention to limit beneficial agent transfer by diffusion and enhance control of the delivery profile of the beneficial agent by controlling the bioerosion characteristics of the polymer. The preferred compositions allow beneficial agent to be loaded into the interior of the polymer at levels that are above that required to saturate the beneficial agent in water, thereby facilitating zero order release of beneficial agent if desired. Additionally, the preferred compositions may provide viscous gels that have a glass transition temperature that is less than 37°C, such that the gel remains non-rigid for a period of time after implantation of 24 hours or more.

The solvent or solvent mixture is capable of dissolving the polymer to form a viscous gel that can maintain compressed particles of the beneficial agent dispersed within it and isolated from the environment of use prior to release. The compositions of the present invention provide implants having a low burst index. Water uptake may be controlled in the microenvironment of the beneficial agent by using compressed particulates of the beneficial agent as described herein and in the macroenvironment of the implant by the use of a solvent or component solvent mixture that solublizes or plasticizes the polymer but substantially restricts bulk uptake of water into implant.

The desirable limit on the amount of beneficial agent released in the first 24 hours that is either desired or required will depend on circumstances such as the overall duration of the delivery period, the therapeutic window for the beneficial agent, potential adverse consequences due to overdosing, cost of beneficial agent, and the type of effect desired, e.g., systemic or local. Preferably, 20% or less of the beneficial agent will be released in the first 24 hours after implantation, where the percentage is based on the total amount of beneficial agent to be delivered over the duration of the delivery period. Typically, higher percentages of release in the first 24 hours can be tolerated if the duration of the delivery period is relatively short, e.g., less than 7-14 days, or if the beneficial agent has a wide therapeutic window with little likelihood of side effects, or if the beneficial agent acts locally.

The compositions of the present invention intended for systemic delivery may provide a gel composition having a burst index of 8 or less, preferably 6 or less, more preferably 4 or less and most preferably 2 or less. Compositions intended for local delivery of beneficial agent are formed in the same manner as those intended for systemic use. However, because local delivery of beneficial agent to a subject will not result in detectable plasma levels of beneficial agent, such systems have to be characterized by a percentage of beneficial agent released in a predetermined initial period, rather than a burst index as defined herein. Most typically, that period will be the first 24 hours after implantation and the percentage will be equal to the amount by weight of the beneficial agent released in the period (e.g. 24 hours) divided by the amount by weight of the beneficial agent intended to be delivered in the duration of the delivery period; multiplied by the number 100. Compositions of the present invention may have initial bursts of 20% or less, preferably 15% or less, most preferably 10% or less, for most applications. Implant systems having initial bursts of 5% or less often are preferred.

The solvent or solvent mixture is typically present in an amount of from about 95 to about 20% by weight of the viscous gel, i.e., the combined weight of the polymer and the solvent. It may be preferably present in an amount of from about 70 to about 30% by weight and often 60-40% by weight of the viscous gel, i.e., the combined weight of the polymer and the solvent. The viscous gel formed by mixing the polymer and the solvent typically exhibits a viscosity of from about 1,000 to about 2,000,000 poise, preferably from about 5,000 to about 50,000 poise measured at a 1.0 sec⁻¹ shear rate and 25°C using a Haake Rheometer at about 1-2 days after mixing is completed.

Mixing the polymer with the solvent can be achieved with conventional low shear equipment such as a Ross double planetary mixer for from about 10 minutes to about 12 hours, often about 1-4 hours, although shorter and longer periods may be chosen by one skilled in the art depending on the particular physical characteristics of the composition being prepared. Gentle heating of the polymer/solvent mixture, e.g., up to about 40° C, may be applied to reduce the time for dissolution of the polymer.

Since it is often desirable to administer the implant as an injectable composition, a countervailing consideration when forming implants that are viscous gels is that the polymer/solvent/beneficial agent composition have sufficiently low viscosity in order to permit it to be forced through a small diameter, e.g., 18-20 gauge needle. If necessary, adjustment of viscosity of the gel for injection can be accomplished with emulsifying agents as described herein. Yet, such compositions should have adequate dimensional stability so as to remain localized and be able to be removed if necessary. The particular gel or gel-like compositions of the present invention satisfy such requirements.

If the polymer composition is to be administered as an injectable gel, the level of polymer dissolution will need to be balanced with the resulting gel viscosity, to permit a reasonable force to dispense the viscous gel from a needle, and the potential burst effect. Highly viscous gels enable the beneficial agent to be delivered without exhibiting a significant burst effect, but may make it difficult to dispense the gel through a needle. In those instances, an emulsifying agent may optionally be added to the composition. Also, since the viscosity may generally be lowered as the temperature of the composition increases, it may be advantageous in certain applications to reduce the viscosity of the gel by heating to provide a more readily injectable composition. Additionally or alternatively, the gel may be mixed prior to injection to shear the gel and reduce the viscosity which may have increased during storage.

The shear thinning characteristics of the depot gel compositions of the present invention are usually favorable and generally allow the gels to be readily injected into an animal including humans using standard gauge needles without requiring undue dispensing pressure.

When used, the emulsifying agent typically is present in an amount ranging from about 5 to about 80%, preferably from about 20 to about 60% and often 30 to 50% by weight based on the amount of the injectable depot gel composition, that is the combined amounts of polymer, solvent, emulsifying agent and beneficial agent. Emulsifying agents include, for example, solvents that are not fully miscible with the polymer solvent or solvent mixture. Illustrative emulsifying agents are water, alcohols, polyols, esters, carboxylic acids, ketones, aldehydes and mixtures thereof. Preferred emulsifying agents are alcohols, propylene glycol, ethylene glycol, glycerol, water, and solutions and mixtures thereof. Especially preferred are water, ethanol, and isopropyl alcohol and solutions and mixtures thereof. The type of emulsifying agent affects the size of the dispersed droplets. For instance, ethanol will provide droplets that have average diameters that can be on the order of ten times larger than the droplets obtained with an isotonic saline solution containing 0.9% by weight of sodium chloride at 21 °C.

Since the implant systems of the present invention preferably are formed as viscous gels, the means of administration of the implants is not limited to injection, although that mode of delivery may often be preferred. Where the implant will be administered as a leave-behind product, it may be formed to fit into a body cavity existing after completion of surgery or it may be applied as a flowable gel by brushing or palleting the gel onto residual tissue or bone. Such applications may permit loading of beneficial agent in the gel above concentrations typically present with injectable compositions.

To form a suspension or dispersion of particles of the beneficial agent in the viscous gel formed from the polymer and the solvent, any conventional low shear device can be used such as a Ross double planetary mixer at ambient conditions. In this manner, efficient distribution of the beneficial agent can be achieved substantially without degrading the beneficial agent.

The beneficial agent is typically dissolved or dispersed in the composition in an amount of from about 1 to about 50% by weight, preferably in an amount of from about 5 to about 30% and often 10 to 20% by weight of the combined amounts of the polymer, solvent and beneficial agent. Depending on the amount of beneficial agent present in the composition, one can obtain different release profiles and burst indices. More specifically, for a given polymer and solvent, by adjusting the amounts of these components and the amount of the beneficial agent, one can obtain a release profile that depends more on the degradation of the polymer than the diffusion of the beneficial agent from the composition or vice versa. In this respect, at lower beneficial agent loading rates, one generally obtains a release profile reflecting degradation of the polymer wherein the release rate increases with time. At higher loading rates, one generally obtains a release profile representative of diffusion of the beneficial agent wherein the release rate decreases with time. At intermediate loading rates, one obtains combined release profiles so that if desired, a substantially constant release rate can be attained. In order to minimize burst, loading of beneficial agent on the order of 30% or less by weight of the overall gel composition, i.e., polymer, solvent and beneficial agent, is preferred, and loading of 20% or less is more preferred.

Release rates and loading of beneficial agent will be adjusted to provide for therapeutically-effective delivery of the beneficial agent over the intended sustained delivery period. The beneficial agent may be present in the polymer gel at concentrations that are above the saturation concentration of beneficial agent in water to provide a drug reservoir from which the beneficial agent is dispensed. While the release rate of beneficial agent depends on the particular circumstances, such as the beneficial agent to be administered, release rates on the order of from about 0.01 micrograms/day to about 100 milligrams/day, preferably from about 0.1 to about 10 milligrams/day, for periods of from about 7 to about 90 days can be obtained. Greater amounts may be delivered if delivery is to occur over shorter periods. Generally, a higher release rate is possible if a greater burst can be tolerated. In instances where the gel composition is surgically implanted, or used as a "leave behind" depot when surgery to treat the disease state or another condition is concurrently conducted, it is possible to provide higher doses that would normally be administered if the implant was injected. Further, the dose of beneficial agent may be controlled by adjusting the volume of the gel implanted or the injectable gel injected.

Other components may be present in the gel composition, to the extent they are desired or provide useful properties to the composition, such as polyethylene glycol, hydroscopic agents, stabilizing agents, pore forming agents, and others. Various stabilizing agents are described in U.S. Patent Nos. 5,654,010 and 5,656,297 which are incorporated herein by reference. While it is generally considered that the practice of the present invention will avoid the need for stabilizing agents for the beneficial agent in the composition, there may be instances where such agents may be advantageous when employed in combination with the components of the compositions of the present invention.

Pore forming agents include biocompatible materials that when contacted with body fluids dissolve, disperse or degrade to create pores or channels in the polymer matrix. Typically, organic and non-organic materials that are water soluble such as sugars (e.g., sucrose, dextrose), water soluble salts (e.g., sodium chloride, sodium phosphate, potassium chloride, and sodium carbonate), water soluble solvents such as N-methyl-2-pyrrolidone and polyethylene glycol and water soluble polymers (e.g., carboxmethylcellulose, hydroxypropylcellulose, and the like) can conveniently be used as pore formers. Such materials may be present in amounts varying from about 0.1 % to about 100% of the weight of the polymer, but will typically be less than 50% and more typically less than 10-20% of the weight of polymer.

To further understand the various aspects of the present invention, reference may be made to Figure 1, wherein a general process flow chart is illustrated for preparing the compositions of the invention. The processes of the invention will be described particularly with respect to hGH (human growth hormone) or lysozyme as the beneficial agents, stearic acid as the agent exhibiting a characteristic of low solubility in water, and PLGA as the biocompatible carrier, as representative examples. However, the processes should be understood to have general applicability to the preparation of compositions of the present invention utilizing other materials as described herein, with appropriate modification as will be apparent to one skilled in the art.

The process flow diagram illustrated in Figure 1 outlines the various steps that may be involved in the production of finished product, i.e. preloaded syringes containing an injectable hydrophobic agent/active agent/polymer depot composition. The flow diagram may be read in conjunction with the following description.

A amount of hydrophobic agent, e.g. stearic acid, appropriate for the batch size being run, stored at station A is transferred in step 1 to a grinding or milling apparatus at station B. Grinding is an optional step that may not be required depending on the particle size of the hydrophobic agent that is obtained as a source material. The ground hydrophobic agent is then transferred in step 2 to a sterilization station C, where the pulverized hydrophobic agent is sterilized using conventional radiation sterilizing equipment. Gamma radiation emitted from cobalt-60 or cesium-137 may be utilized. A radiation dose of about 16 Kilogray (KGy) from a cobalt-60 source has been found to be satisfactory.

The sterilized hydrophobic agent powder is transferred in step 3 to a mixing chamber at station J, which also receives sterilized active agent, e.g., human growth hormone or lysozyme, from station I, via step 4. Mixing may be by hand if quantities are small or by means of a V-blender or other conventional mixing apparatus for larger quantities. The mixed protein/hydrophobic agent blend is then transferred in step 5 to a compaction station K at which the powder blend is compacted by tabletting, roller compaction or extrusion by conventional means as described elsewhere herein. The compacted material then is transferred to a grinder or milling apparatus at station L where it is ground into particulates, and sieved through screens at station M. Generally, those particulates that pass through a 70 mesh screen and are retained on a 400 mesh screen are utilized to prepare the dispersed particulate/polymer composition. Particulates collected on a 70 mesh screen may be recycled to the grinder at station L, and particulates passing through a 400 mesh screen are transferred in a step 7 to disposal or recycled. The sized, collected particles are transferred in a step 6 to a mixing vessel at station N, to which a sterilized mixture of polymer and solvent prepared as below is also transferred.

An amount of solvent, e.g. benzyl benzoate, stored at station D, and an amount of polymer, e.g. PLGA, stored at station E are transferred via steps 8 and 9, respectively to a mixing vessel at station F. The mixing vessel may be any suitable conventional mixing apparatus, such as a V-blender or a Wharing blender. Initial mixing generally takes place at room temperature over a period of hours as described elsewhere herein. The initially mixed material may be transferred to a temperature controlled mixing vessel at station G where mixing continues at an elevated temperature, e.g. 35°C-40°C, until the polymer solution is a homogenous mass. The mixed polymer/solvent gel is transferred via step 11 to station H at which it is sterilized in a manner as described herein with respect to the hydrophobic agent and transferred via step 12 to a mixing vessel at station N. There the polymer/solvent and protein/hydrophobic agent particulates are mixed to uniformly disperse the particulates throughout the polymer carrier composition.

Sterile syringes from station O are transferred to the aseptic manufacturing area in step 14 to a station P where the syringes are aseptically filled with the desired volume of the protein/hydrophobic agent/polymer gel composition. Filled syringes are transferred in step 15 to a primary packaging station Q, and then the packaged syringes are transferred from the aseptic manufacturing area in step 16 to a secondary packaging and labeling station R. The labeled and bulk packaged syringes are transferred in a step 17 to a final station S for storage and shipment.

In a particular application of the general process, an appropriate quantity of stearic acid (Sigma - Aldrich Chemical Company) is ground or milled to a powder using a mortar and pestle or automated grinder or mill, if not received in a suitable powdered state that will permit intimate mixing of stearic acid with the beneficial agent. Smaller sized stearic acid particles generally facilitate better mixing with the beneficial agent. Preferably, the stearic acid, which is a mixture of stearic acid and palmitic acid, has a stearic acid content of not less than 40% and the sum of the two acids being not less than 90% by weight. Higher percentages of stearic acid in the stearic acid/palmitic acid mixtures are preferred. The stearic acid powder is sterilized using cobalt 60 at a dose of 16 kGy at a rate of 1 kilogray per hour (kGy/hr). Alternatively, the stearic acid may be sterilized by melting, followed by microfiltration.

Gel vehicle may be prepared as described in Example 1 below, and sterilized prior to mixing with the stearic acid/hGH compressed particulates. Lyophilized hGH and lysozyme particles may be prepared as described in Examples 2 and 3 below, respectively. Typically, equal quantities by weight of the protein and the stearic acid, e.g, 10% by weight of the overall composition for each component, are mixed as dry powders. Mixing may be by hand if quantities are small or with a V-blender or other conventional mixing apparatus for larger quantities. The mixture of beneficial agent and stearic acid is then pelletized in a Carver press at 10,000-12,000 psi using a 13 mm diameter die for about 5 minutes. Other conventional tableting presses may be used in place of the Carver press for larger scale operation.

After the protein/stearic acid mixture has been compressed, it is granulated or milled to a powder in a mortar and pestle or conventional, larger-scale milling apparatus. The granulated mixture is sieved through a 212 micron sized screen and collected on a 53 micron sized screen. Those sizes correspond generally to a sieve #70 and sieve #400. Particles that pass through the 400 mesh screen are discarded or recycled.

In another process, the stearic acid may be added to a solution of the beneficial agent, e.g., the hGH diafiltered solution prepared in Example 2, prior to a lyophilization step to produce lyophilized particles of beneficial agent/stearic acid. The lyophilized particles are then compressed, granulated and sieved as described above to provide compressed particulates of the beneficial agent/stearic acid mixture.

Compressed particulates of the beneficial agent/stearic acid mixture collected from the 400 mesh screen are mixed with the gel vehicle in a Lightning overhead mixer for approximately 5-10 minutes, or until the mixture otherwise approaches or reaches homogeneity. The time frame is not presently considered critical and will depend in part on the nature of the mixing apparatus employed. For example, if a Ross or double planetary mixer is employed for larger scale operations, the mixing time may need to be longer.

Following mixing of the compressed particulates of beneficial agent/stearic acid with the gel vehicle, the combined mixture is loaded into sterilized syringes under aseptic filling conditions to produce a final product that when packaged to maintain sterility may be used directly without further sterilization at the site of application.

Utilizing the ratios of materials described in the following examples, a product comprising a viscous gel in which the compressed particulates of beneficial agent/stearic acid are dispersed may be directly injected into an application site in a subject. Alternatively, implants comprising the viscous gels or more rigid implants formed with lesser amounts of solvents may be formed outside of the body of a subject and implanted using surgical procedures as appropriate.

### Example 1 - Gel Vehicle Preparation

A glass vessel is tared on a Mettler PJ3000 top loader balance. Poly (D,L-lactide-co-glycolide) 50:50 RESOMER® RG502 (PLGA-502) is weighed into the glass vessel. The glass vessel containing PLGA-502 is tared and the corresponding solvent is added. Amounts expressed as percentages for various polymer/solvent combinations are set forth in Table 1 below. The polymer/solvent mixture is manually stirred with a stainless steel square-tip spatula, resulting in a sticky amber paste-like substance containing white polymer particles. The vessel containing the polymer/solvent mixture is sealed and placed in a temperature controlled incubator equilibrated to 37°C - 39°C. The polymer/solvent mixture is removed from the incubator when it appears to be a clear amber homogeneous gel. Incubation time intervals may range from 1 to 4 days, depending on solvent and polymer type and solvent and polymer ratios. Additional depot gel vehicles are prepared with the following polymers: Poly (D,L-lactide-co-glycolide) 50:50 RESOMER® L104, PLGA-L104, code no. 33007, Poly (D,L-lactide-co-glycolide) 50:50 RESOMER® RG206, PLGA-206, code no. 8815, Poly (D,L-lactide-co-glycolide) 50:50 RESOMER® RG502, PLGA-502, code 0000366, Poly (D,L-lactide-co-glycolide) 50:50 RESOMER® RG502H, PLGA-502H, code no. 260187, Poly (D,L-lactide-co-glycolide) 50:50 RESOMER® RG503, PLGA-503, code no. 0080765, Poly (D,L-lactide-co-glycolide) 50:50 RESOMER® RG506, PLGA-506, code no. 95051, Poly (D,L-lactide-co-glycolide) 50:50 RESOMER® RG755, PLGA-755, code no. 95037, (Boehringer Ingelheim Chemicals, Inc., Petersburg, VA), and the following solvents or mixtures: glyceryl triacetate (Eastman Chemical Co., Kingsport, TN), benzyl benzoate ("BB"), ethyl benzoate ("EB"), methyl benzoate ("MB"), triacetin ("TA"), and triethyl citrate ("TC") (Aldrich Chemical Co., St Louis, MO). When solvent combinations were used, for example 20% triacetin and 80% benzyl benzoate, the solvent mixture was directly added to the pre-weighed dry polymer. Typical polymer molecular weights were in the range of 14,400 - 39,700 (M_{w}) [6,400-12,200 (Mₙ)]. Representative gel vehicles are described in Table 1 below.

### Example 2 - hGH Particle Preparation

Human growth hormone (hGH) particles (optionally containing zinc acetate) were prepared as follows:
hGH solution (5 mg/ml) solution in water (BresaGen Corporation, Adelaide, Australia) is concentrated to 10 mg/mL using a Concentration/Dialysis Selector diafiltering apparatus. The diafiltered hGH solution is then washed with 5 times volume of tris or phosphate buffer solution (pH 7.6). Particles of hGH are then formed by spray drying or lyophilization using conventional techniques. Phosphate buffer solutions (5 or 50 mM) containing hGH (5 mg/mL) (and optionally various levels of zinc acetate (0 to 30 mM) when Zn complexed particles are prepared) are spray-dried using a Yamato Mini Spray dryer set at the following parameters:

| **Spray Dryer Parameter** | **Setting** |
|---|---|
| Atomizing Air | 2 psi |
| Inlet Temperature | 120°C |
| Aspirator Dial | 7.5 |
| Solution Pump | 2-4 |
| Main Air Valve | 40-45 psi |

hGH particles having a size range between 2 - 100 microns are obtained. Lyophilized particles are prepared from tris buffer solutions (5 or 50 mM: pH 7.6) containing hGH (5 mg/mL) using a Durastop µP Lyophilizer in accordance with the following freezing and drying cycles:

| | |
|---|---|
| **Freezing Cycle** | Ramp down at 2.5 C/min to -30 C and hold for 30 minutes |
| | Ramp down at 2.5 C/min from -30 C to -50C and hold for 60 minutes |
| **Drying Cycle** | Ramp up at 0.5 C/min to 10 C and hold for 960 min |
| | Ramp up at 0.5 C/min to 20 C and hold for 480 min |
| | Ramp up at 0.5 C/min to 25 C and hold for 300 min |
| | Ramp up at 0.5 C/min to 30 C and hold for 300 min |
| | Ramp down at 0.5 C/min to 5 C and hold for 5000 min |

hGH particles having a size range between 2 - 100 microns are obtained.

### Example 3

Lysozyme particles are prepared by spray drying 50% sucrose and 50% chicken lysozyme (on a dry weight basis) using the procedure described in Example 2. Those particles are mixed with stearic acid, palmitic acid, and myristic acid, respectively, in the manner described above to produce compressed particulates comprising a mixture of lysozyme and the corresponding fatty acid having particle sizes between about 40 µm and 200 µm. Two stearic acid batches had mean particles sizes of 65 µm and 85 µm, respectively;, two palmitic acid batches had mean particle sizes of 80 µm and 76 µm, respectively; and a myrstic acid batch had a mean particle size of 74 µm.

**Table 1: Gel Vehicles**

| Solvent/ Polymer | Solvent | Polymer | Amount Solvent | Amount Polymer | Gel Weight | Ratio |
|---|---|---|---|---|---|---|
| 50/50 | BB | PLGA-502 | 5g | 5g | 10g | 1.0 |
| 50/50 | TA / BB Mixture | PLGA-502 | 5 g | 5 g | 10g | 1.0 |
| 60/40 | TA / BB Mixture | PLGA-502 | 6 g | 4 g | 10g | 1.5 |
| 70/30 | TA / BB Mixture | PLGA-502 | 7 g | 3 g | 10g | 2.3 |
| 80/20 | TA / BB Mixture | PLGA-502 | 8g | 2g | 10g | 4.0 |
| 50/50 | EB | PLGA-502 | 5g | 5g | 10g | 1.0 |
| 50/50 | TA / EB Mixture | PLGA-502 | 5 g | 5 g | 10g | 1.0 |
| 50/50 | BB | PLGA-502 | 25g | 25g | 50g | 1.0 |
| 55/45 | BB | PLGA-502 | 27.5g | 22.5g | 50g | 1.2 |
| 50/50 | BB | PLGA-502 | 50 g | 50 g | 100 g | 1.0 |
| 50/50 | TA / BB Mixture | PLGA-502 | 50 g | 50 g | 100 g | 1.0 |
| 50/50 | BB | PLGA-502H | 5 g | 5 g | 10 g | 1.0 |
| 50/50 | BB | PLGA-503 | 50 g | 50 g | 100 g | 1.0 |

### Drug Loading

Compressed particulates comprising beneficial agent/stearic acid prepared as above are added to a gel vehicle in an amount of 10 - 20 % by weight and blended manually until the dry powder is wetted completely. Then, the milky light yellow particle/gel mixture is thoroughly blended by conventional mixing using a Caframo mechanical stirrer with an attached square-tip metal spatula. Final homogenous gel formulations were transferred to 3, 10 or 30 cc disposable syringes for storage or dispensing.

A representative number of implantable gels were prepared in accordance with the foregoing procedures and tested for in vitro release of beneficial agent as a function of time and also in in vivo studies in rats to determine release of the beneficial agent as determined by blood plasma concentrations of beneficial agent as a function of time.

As can be see with reference to Figure 2, lysozyme that is not present in a gel vehicle as a compressed mixture with stearic acid or palmitic acid is released from the gel much more rapidly and to a greater extent when measured in a USP dissolution bath containing a phosphate buffer at 100 rpm. The percent lysozyme in the non-compressed state that is released is on the order of 3-4 times greater than that released from the gel vehicles that contain particulates formed of a compressed mixture of lysozyme and stearic acid and palmitic acid, respectively. The advantageous effects of the compressed particulates are also demonstrated in a test for the dissolution of the particulates themselves as described in Figure 3 where practically complete dissolution of lysozyme particles that are not present in a form of a compressed particulate with stearic acid or palmitic acid is illustrated. Similar results are illustrated in Figure 4 for uncompressed lysozyme particles and compressed particulates of lysozyme with stearic acid, myrstic acid and palmitic acid.

The in vivo release of lysozyme from PLGA 502/benzyl benzoate (50-50) gels prepared as above and containing 10% by weight lyophilized hGH particles and compressed hGH/stearic acid particles where the stearic acid is present in one case in an equal amount to the hGH on a weight basis (designated as "low") and in another case in an amount twice the weight of the hGH (designated as "high") is illustrated in Figure 5. The concentration of hGH in blood serum of a rat (normalized for body weight) is plotted against time after implantation in days. As shown, the uncompressed hGH particles exhibit a very high initial burst of hGH after implantation, so much so that most of the protein is released from the implant within a single day of implantation. In sharp contrast, both formulations of hGH with stearic acid exhibit a very low initial burst of protein and provided sustained release of hGH from the implant for more than 14 days.

Figure 6 illustrates the advantageous effects of the combination of stearic acid to control the microenvironment about an hGH particle and the macroenvironment of the PLGA implant using an ester of benzoic acid, namely ethyl benzoate and benzyl benzoate, thus inhibiting gross intake of water into the implant after implantation. As can be readily discerned, the release of hGH from compressed hGH/stearic acid particles in a PLGA-502 implant prepared with ethyl benzoate or benzyl benzoate as solvents for the polymer exhibits a low initial burst and a sustained release of hGH over time.

The present invention is described and characterized by one or more of the following technical features and/or characteristics, either alone or in combination with one or more of the other features and characteristics:
a composition comprising a biocompatible carrier and particulates comprising a compressed mixture of an active agent and, optionally a dissolution rate modulating agent or an agent exhibiting a characteristic of low solubility in water, the particulates being dispersed within the carrier; a composition comprising a biocompatible carrier and particulates comprising a compressed mixture of an active agent and a dissolution rate modulating agent, the particulates being dispersed within the carrier; a composition comprising a biocompatible carrier and particulates comprising a compressed mixture of an active agent and an agent exhibiting a characteristic of low solubility in water, the particulates being dispersed within the carrier; a composition wherein the agent exhibiting the characteristic of low solubility in water is hydrophobic and the carrier is a biocompatible gel; a composition wherein the hydrophobic agent comprises a pharmaceutically acceptable oil, fat, fatty acid, fatty acid ester, wax or derivative thereof that exhibits the hydrophobic characteristic; a composition wherein the hydrophobic agent comprises a C₁₆ - C₂₄ fatty acid, or an ester or pharmaceutically-acceptable salt thereof, or a mixture of any of the foregoing; a composition wherein the hydrophobic agent comprises a mixture of stearic acid and palmitic acid; a composition wherein the stearic acid and the palmitic acid together constitute at least 90% by weight of the fatty acids of the hydrophobic agent and the stearic acid constitutes at least 40% by weight of the fatty acids of the hydrophobic agent; a composition wherein the stearic acid and the palmitic acid together constitute at least 96% by weight of the fatty acids of the hydrophobic agent and the stearic acid constitutes at least 90% by weight of the fatty acids of the hydrophobic agent; a composition wherein the particulates comprise a powder; a composition wherein the powder has a particle size such that 90% passes through a 50 mesh screen and is retained on a 400 mesh screen; a composition wherein the powder has a particle size such that the powder passes through a 70 mesh screen and is retained on a 400 mesh screen; a composition wherein the particulates have a size between 0.1-500 microns; a composition wherein the particulates have a size between 0.1-500 microns; a composition wherein the particulates have a size between 30-400 microns; a composition wherein the active agent is water soluble; a composition wherein the active agent is selected from the group consisting of DNA, cDNA, proteins, peptides and fragments and derivatives thereof; a composition wherein the carrier comprises a polymer selected from the group consisting of polylactic acid, polyglycolic acid and poly(lactide-co-glycolic) acid and a solvent comprising an alkyl or aralkyl ester of benzoic acid; a composition wherein the active agent is human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor or filgrastim; a composition wherein the polymer is poly(lactide-co-glycolic) acid and the solvent is benzyl benzoate; a composition wherein the polymer is poly(lactide-co-glycolic) acid and the solvent is ethyl benzoate; a composition comprising a bioerodible gel comprising a polymer selected from polylactic acid, polyglycolic acid, and poly(lactide-co-glycolic) acid, a solvent selected from an alkyl or aralkyl ester of benzoic acid, and particulates comprising a compressed mixture of an active agent and an agent exhibiting a characteristic of low solubility in water selected from the group consisting of a pharmaceutically acceptable oil, fat, fatty acid, fatty acid ester, wax, a derivative thereof, or a mixture of the foregoing, the particulates being dispersed within the gel; a process for the preparation of an implantable carrier having dispersed therein an active agent which comprises forming a compressed body of an active agent, optionally mixed with a dissolution rate modulator or an agent exhibiting a characteristic of low solubility in water, crushing the body to form compressed particulates comprising the active agent, optionally mixed with a dissolution rate modulator or an agent exhibiting a characteristic of low solubility in water, and dispersing the compressed particulates throughout the carrier; a process wherein the active agent is water soluble and the agent exhibiting a characteristic of low solubility in water is hydrophobic; a process wherein the active agent is a protein or polypeptide and the hydrophobic agent is stearic acid, palmitic acid or myrstic acid; a process wherein the protein is human growth hormone and the hydrophobic agent is stearic acid; a process wherein the active agent is selected from the group consisting of cDNA, DNA, proteins, peptides and fragments and derivatives thereof; a process wherein the active agent is selected from human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor or filgrastim.

The above-described exemplary embodiments are intended to be illustrative in all respects, rather than restrictive, of the present invention. Thus the present invention is capable of many variations in detailed implementation that can be derived from the description contained herein by a person skilled in the art. All such variations and modifications are considered to be within the scope and spirit of the present invention.

## Claims

1. A composition comprising a carrier and particulates comprising a compressed mixture of an active agent and an agent exhibiting a characteristic of low solubility in water, the particulates being dispersed within the carrier.

2. The composition of claim 1 wherein the agent exhibiting the characteristic of low solubility in water is hydrophobic and the carrier is a biocompatible gel.

3. The composition of claim 1 wherein the hydrophobic agent is selected from the group consisting of pharmaceutically acceptable oil, fats, fatty acids, fatty acid esters, waxes and mixtures and derivatives thereof that exhibit the hydrophobic characteristic.

4. The composition of claim 3 wherein the hydrophobic agent is selected from the group consisting of C₁₆ - C₂₄ fatty acids, esters and pharmaceutically-acceptable salts thereof, and mixtures of the foregoing.

5. The composition of claim 4 wherein the hydrophobic agent comprises a mixture of stearic acid and palmitic acid.

6. The composition of claim 5 wherein the stearic acid and the palmitic acid together constitute at least 90% by weight of the fatty acids of the hydrophobic agent and the stearic acid constitutes at least 40% by weight of the fatty acids of the hydrophobic agent.

7. The composition of claim 6 wherein the stearic acid and the palmitic acid together constitute at least 96% by weight of the fatty acids of the hydrophobic agent and the stearic acid constitutes at least 90% by weight of the fatty acids of the hydrophobic agent.

8. The composition of claim 1 wherein the particulates comprise a powder.

9. The composition of claim 1 wherein the powder has a particle size such that 90% passes through a 50 mesh screen and are retained on a 400 mesh screen.

10. The composition of claim 1 wherein the active agent is water soluble.

11. The composition of claim 10 wherein the active agent is selected from the group consisting of DNA, cDNA, proteins, peptides and fragments and derivatives thereof.

12. The composition of claim 10 wherein the carrier comprises a polymer selected from the group consisting of polylactic acid, polyglycolic acid and poly(lactide-co-glycolic) acid and a solvent comprising an alkyl or aralkyl ester of benzoic acid.

13. The composition of claim 12 wherein the active agent is selected from the group consisting of human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor and filgrastim.

14. The composition of claim 13 wherein the polymer is poly(lactide-co-glycolic) acid and the solvent is benzyl benzoate.

15. The composition of claim 14 wherein the polymer is poly(lactide-co-glycolic) acid and the solvent is ethyl benzoate.

16. A composition comprising: (a) a bioerodible gel comprising a polymer selected from the group consisting of polylactic acid, polyglycolic acid, and poly(lactide-co-glycolic) acid; (b) a solvent selected from the group consisting of an alkyl or aralkyl ester of benzoic acid; and (c) particulates dispersed within the gel, said particulates comprising a compressed mixture of an active agent and an agent exhibiting a characteristic of low solubility in water selected from the group consisting of pharmaceutically acceptable oils, fats, fatty acids, fatty acid esters, waxes, derivatives thereof, and mixtures of the foregoing.

17. The composition of claim 16 wherein the agent exhibiting the characteristic of low solubility in water is hydrophobic.

18. The composition of claim 17 wherein the hydrophobic agent is selected from the group consisting of C₁₆- C₂₄ fatty acids, esters and pharmaceutically-acceptable salts thereof, and mixtures of the foregoing.

19. The composition of claim 18 wherein the hydrophobic agent comprises a mixture of stearic acid and palmitic acid.

20. The composition of claim 19 wherein the stearic acid and the palmitic acid together constitute at least 90% by weight of the fatty acids of the hydrophobic agent and the stearic acid constitutes at least 40% by weight of the fatty acids of the hydrophobic agent.

21. The composition of claim 20 wherein the stearic acid and the palmitic acid together constitute at least 96% by weight of the fatty acids of the hydrophobic agent and the stearic acid constitutes at least 90% by weight of the fatty acids of the hydrophobic agent.

22. The composition of claim 21 wherein the particulates comprise a powder.

23. The composition of claim 22 wherein the powder has a mean particle size of about 30 microns to about 500 microns.

24. The composition of claim 23 wherein the active agent is water soluble.

25. The composition of claim 24 wherein the active agent is selected from the group consisting of DNA, cDNA, proteins, peptides and fragments and derivatives thereof.

26. The composition of claim 24 wherein the gel comprises poly(lactide-co-glycolic) acid.

27. The composition of claim 24 wherein the active agent is selected from the group consisting of human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor and filgrastim.

28. The composition of claim 27 wherein the solvent is benzyl benzoate and the active agent is human growth hormone.

29. The composition of claim 27 wherein the solvent is ethyl benzoate and the active agent is human growth hormone.

30. A process for the preparation of an implantable composition comprising a bioerodible carrier having dispersed therein an active agent that comprises forming a compressed body of a mixture of the active agent and an agent exhibiting a characteristic of low solubility in water, crushing the body to form compressed particulates of the mixture of the active agent and the agent exhibiting a characteristic of low solubility in water, and dispersing the compressed particulates throughout the carrier.

31. The process of claim 30 wherein the active agent is water soluble and the agent exhibiting a characteristic of low solubility in water is hydrophobic.

32. The process of claim 31 wherein the active agent is selected from the group consisting of protein and polypeptide and the hydrophobic agent is selected from the group consisting of stearic acid, palmitic acid and myristic acid.

33. The process of claim 32 wherein the protein is human growth hormone and the hydrophobic agent is stearic acid.

34. The process of claim 31 wherein the active agent is selected from the group consisting of cDNA, DNA, proteins, peptides and fragments and derivatives thereof.

35. The process of claim 31 wherein the active agent is selected from the group consisting of human growth hormone, alpha-, beta- or gamma-interferon, erythropoietin, glugacon, calcitonin, heparin, interleukin-1, interleukin-2, Factor VIII, Factor IX, luteinizing hormone, relaxin, follicle-stimulating hormone, atrial natriuretic factor and filgrastim.
